# EUROPEAN PATENT APPLICATION

(11) **EP 3 639 729 A1**
(43) Date of publication of application: **22.04.2020**
(21) Application number: 18200595.9
(22) Date of filing: 16.10.2018
(51) Int. Cl.: A61B 5/00, A61M 25/09, A61B 5/0215, A61B 5/027, H01Q 21/08

(54) **ENERGY TRANSMISSION FOR AN INTERVENTIONAL DEVICE FOR MEDICAL INTERVENTIONS**

(71) Applicant: Koninklijke Philips N.V., 5656 AG Eindhoven (NL)
(72) Inventor: GLEICH, Bernhard, 5656 AE Eindhoven (NL); RAHMER, Jürgen Erwin, 5656 AE Eindhoven (NL)
(74) Representative: de Haan, Poul Erik

(57) **Abstract**

The present invention relates to energy transmission of an interventional device. In order to provide a device with improved handling, a device (10) for medical intervention is provided that comprises an elongated main body (12) and an electronic circuit (14). The elongated main body has a longitudinal axis (16) and comprises a first section (18) and a second section (20) arranged in a consecutive manner along the longitudinal axis. The first section and the second section are mechanically connected to each other forming a continuous mechanical structure. They are also each made from an electrically conductive material. They are further separated from each other by an electrically isolating layer (22). The electronic circuit is attached to the elongated main body and is electrically connecting the first section and the second section for being supplied by the transmitted electric energy. The first section and the second section act as an antenna for transmitting electric energy to/from the electronic circuit.

## Description

### FIELD OF THE INVENTION

The present invention relates to energy transmission for an interventional device, and relates in particular to a guidewire for medical intervention, to a system for medical intervention and to a method for supplying an electronic circuit with energy.

### BACKGROUND OF THE INVENTION

In the medical field, guidewires are used for different types of interventions and examination procedures. A guidewire is inserted into a body of a subject, for example into the vascular structure. This may be done under observation by imaging methods like fluoroscopy. Once the guidewire is placed and the tip has reached the target location, the guidewire can act as guide for further steps, like inserting devices like stents or other prosthesis along the guidewire. During placement of the guidewire, sensors on the guidewire can be used to provide data, for example data useful for navigation within a vascular structure. For example, US 2014187982 A1 describes an intravascular device with sensing components in which wireless communication is provided. However, it has been shown that wire connections for energy supply of the sensors or the like may be cumbersome in handling of the guidewire.

### SUMMARY OF THE INVENTION

There may thus be a need to provide an interventional device, in particular a guidewire with improved handling.

The object of the present invention is solved by the subject-matter of the independent claims; further embodiments are incorporated in the dependent claims. It should be noted that the following described aspects of the invention apply also for the guidewire for medical intervention, for the system for medical intervention and for the method for supplying an electronic circuit with energy.

According to the present invention, a guidewire for medical intervention is provided. The guidewire comprises an elongated main body and an electronic circuit. The elongated main body has a longitudinal axis and comprises a first section. The elongated main body further comprises a second section arranged in a consecutive manner along the longitudinal axis. The first section and the second section are mechanically connected to each other forming a continuous mechanical guide. They are each made from an electrically conductive material. In an example the electrically conductive material comprises metal. Further, they are separated from each other by an electrically isolating layer. The electronic circuit is attached to the elongated main body and is electrically connecting the first section and the second section for being supplied by transmitted electric energy. The first section and the second section act as an antenna for transmitting electric energy to/from the electronic circuit.

This provides a facilitated handling, since the guidewire is showing a reduced number of cable connections, as the energy supply is provided in a wireless manner.

According to an example, the first section and the second section are configured as a passive antenna provided to receive energy supplied in form of electromagnetic radiation that generates a local electric potential between the first section and the second section. The electric potential is supplied as electric energy to the electronic circuit.

According to an example, the first section is provided as a guidewire front tip and the second section is provided as a guidewire main shaft portion for an at least partial insertion into a body of a subject.

According to an example, for the mechanical connection, one of the first section and the second section comprises a front-end reception, and the other one of the first section and the second section comprises a front-end extension inserted into the reception.

According to an example, the electronic circuit is provided as a component of at least one of the group of: i) a pressure sensor, ii) a flow sensor and iii) a rectifier.

According to the present invention, also a system for medical intervention is provided. The system comprises a generator for generating electromagnetic radiation and a guidewire according to one of the preceding examples. The generator is configured to generate electromagnetic radiation to be received by the guidewire. The electronic circuit is supplied by electric energy transferred from the received electromagnetic radiation.

According to an example, the generator is configured to generate electromagnetic radiation at an operation frequency that approximately matches with a resonance frequency of the antenna formed by the first section and the second section.

According to the present invention, also a method for supplying an electronic circuit with energy is provided. The method comprises the following steps:
a) providing a guidewire for a medical intervention; wherein the guidewire comprises an elongated main body and an electronic circuit; wherein the elongated main body has a longitudinal axis and comprises a first section and a second section arranged in a consecutive manner along the longitudinal axis; wherein the first section and the second section are mechanically connected to each other forming a continuous guide, and are each made from an electrically conductive material, and are separated from each other by an electrically isolating layer such that the first section and the second section act as an antenna for transmitting electric energy to/from the electronic circuit; wherein the electronic circuit is attached to the elongated main body; and wherein the electronic circuit is electrically connecting the first section and the second section;
b) generating electromagnetic radiation;
c) receiving the electromagnetic radiation by the antenna formed by the first section and the second section and generating an electric potential; and
d) supplying the generated electric potential to the electronic circuit for operating the electronic circuit.

According to an aspect, an enlarged antenna structure is provided. The large antenna is generated by splitting the guidewire in two sections by introducing an insulating connection structure. The high frequency wave travelling on the guidewire produces a relatively large voltage difference at this structure. The rectifying chip (and possibly the whole sensor chip) is placed near the insulating piece and is electrically connected to both sides of the guidewire. The rectified power is either used locally or transmitted to a sensor component, located on the distal portion of the elongate body, by conventional means like wires. The sensor component or sensor element is one of an imaging sensor, a physiological data measurement sensor. In an example, the construction reduces the power requirement by 10 to 20 dB. Hence, the power required on a guidewire is reduced. This supports to reduce power requirements. To form the antenna, the main body is electronically, i.e. conductively, separated into two portions, but the mechanical "one-piece" characteristic of the main body is maintained.

In alternative embodiments of the device, system and methods the guidewire is substituted by one of an interventional device such as intravascular catheter, interventional needle, implantable device for providing physiological measurement data or treatment.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 shows a schematic cross section through an example for a guidewire for medical intervention.
Fig. 2 shows a schematic setup of an example of a system for medical intervention.
Fig. 3 shows steps of an example of a method for supplying an electronic circuit on a guidewire for medical intervention with energy.

### DETAILED DESCRIPTION OF EMBODIMENTS

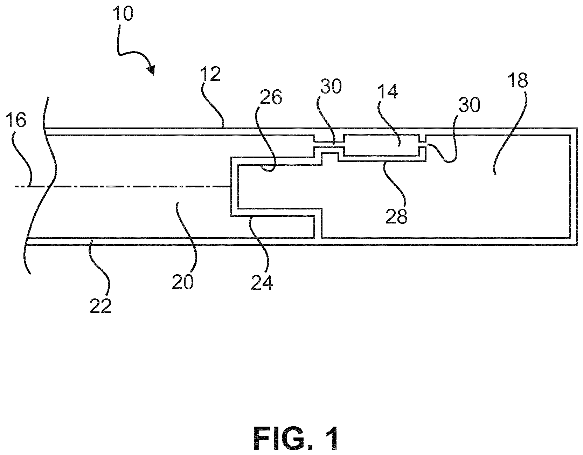

Fig. 1 shows a cross section in longitudinal direction of the guidewire 10 for medical intervention. The guidewire 10 comprises an elongated main body 12 and an electronic circuit 14. The elongated main body 12 has a longitudinal axis 16 and comprises a first section 18 and a second section 20 arranged in a consecutive manner along the longitudinal axis. The first section 18 and the second section 20 are mechanically connected to each other forming a continuous mechanical guide. The first section 18 and the second section 20 are each made from an electrically conductive material. The electrically conductive material can be metal or doped polymer. The first section 18 and the second section 20 are further separated from each other by an electrically isolating layer 22. The electronic circuit 14 is attached to the elongated main body 12 and is electrically connecting the first section 18 and the second section 20 for being supplied by the transmitted electric energy. The first section 18 and the second section 20 act as an antenna for transmitting electric energy to/from the electronic circuit. The electronic circuit may comprise at least one sensor element. In alternative embodiment the sensor element, not shown, is located on the distal portion of the elongated main body, for example on the first section 18.

In an example, the first section 18 and the second section 20 are made from metal. As indicated, the guidewire is made from metal and insulation is provided enclosing the guidewire. Further, the metal of the guidewire is split into two sections, namely the first section 18 and the second section 20, by (polymer) insulation material.

It is noted that the insulation layer around the guidewire is provided as an option. The insulation may also be omitted in examples.

As a result, a separate electric connection for supplying the electronic circuit with energy can be omitted and the handling of the guidewire is thus facilitated.

As an example, for an active CMUT (or piezoresistive) pressure sensor, power can be coupled wirelessly into the guidewire and thus the sensor. Even with small size rectifier chips, a substantial fraction of the high frequency voltage can be supplied into the rectifier. Hence, it is possible to couple high frequencies into the guidewire and hence to feed high frequency voltage to sensor elements.

The term "medical intervention" relates to medical procedures, like examination or treatments or operations, which require interventional devices inserted into a body of a subject.

The term "subject" may also be referred to as individual. The "subject" may further also be referred to as patient, although it is noted that this term does not indicate whether any illness or disease is actually present with the subject.

The term "guidewire" relates to a wire that is used as a guide in medical interventions, e.g. for placement of a device or prosthesis like a stent or the like, or also for placement of a catheter or for guiding operation and placement of an intravascular tool, e.g. an ablation tool.

In an option, as shown, the first section 18 and the second section 20 are configured as a passive antenna provided to receive energy supplied in form of electromagnetic radiation that generates a local electric potential between the first section 18 and the second section 20. The electric potential is supplied as electric energy to the electronic circuit 14. In an example, an electric potential of approximately 1 Volt is provided.

In an example, the elongated main body 12 comprises a first end configured as a front tip for insertion into a subject. The elongated main body 12 comprises a second end configured as handling portion for steering the guidewire inside a subject. The first end is also referred to as distal end. The second end is also referred to as proximal end. The front tip is also referred to as tip and the handling portion as control part.

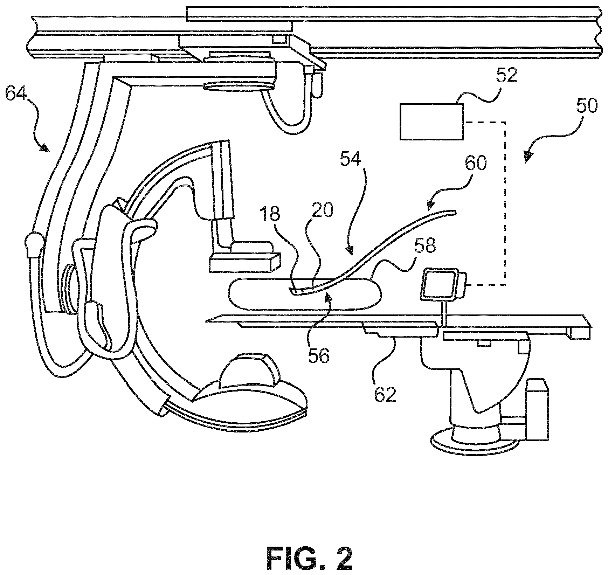

In an example, the first section 18 and the second section 20 are provided in that part of the guidewire that is provided to be inserted into a body of a subject (see also Fig. 2).

In an example, the first section and the second section are provided in the distal part of the guidewire, for example in the distal end or at least close to the distal end, for example less than 50 cm from the distal end, e.g. less than 20 cm, or less than 10 cm.

In an example, the first section 18 and the second section 20 are both provided with a minimum length of 0,1 cm.

In an option, the first section 18 is provided as a guidewire front tip and the second section 20 is provided as a guidewire main shaft portion for an at least partial insertion into a body of a subject.

In an example, the first section 18 is provided with a length of between 0,1 and 6 cm. For example, the first section 18 is provided with a length of 4 cm.

In another example, the second section 20 is provided with a length of between 0,1 and 6 cm. For example, the second section 20 is provided with a length of 4 cm.

In a further example, the second section 20 is provided in a length of the rest of the guidewire, i.e. in a length basically of the whole guidewire.

In a further option, to retain mechanical strength, one section of the wire is inserted into the other to increase the surface area of the glue used for connecting the sections. As shown as an option, for the mechanical connection, one of the first section 18 and the second section 20 comprises a front-end reception 24, and the other one of the first section 18 and the second section 20 comprises a front-end extension 26 inserted into the reception. Optionally, a crimp process is provided that secures the connection further.

The connection of the first section 18 and the second section 20 is provided as a plug connection of a plug coupling. It is noted that the mechanical connection is provided in a fixed manner, i.e. in a permanent manner. The stepped connection enlarges the contact area and thus the active surface for gluing both sections together.

The electric circuit 14 is indicated as electronic chip, placed in a guidewire recess 28 and connected to both sections of the guidewire with connecting sections 30. The length of the short end of the wire, i.e. the section provided at the distal end, does not need to be large. A few millimeters are sufficient to reduce the power requirement considerably and the effect is also shown until about 4 cm of length, depending on the wavelength. The DC power generated in the chip can be used locally, if the pressure sensor is integrated into this chip or sent to a more distal location using DC connection technology, e.g. multiple shells of a conductor. If both ends are reasonably long, e.g. equal or larger than 4 cm, it is also possible to transmit the power not only along the guidewire, but through the patient. For example, lower frequencies, e.g. around 50 MHz, are provided in device operation for a sufficient tissue penetration. In an example, a minimum operation voltage is about 1 Volts or 2 Volts and a maximum length about 8 cm, and the field strength required is 25 V/m resulting in a power dissipation of about 3 W/kg. This power level is permissible for example in MRI operation. For operation in a cathlab procedure, different values are provided.

In any of the examples, the first section and the second section can be provided with an outer isolating layer.

In any of the examples, the electronic circuit 14 is an electronic component. In an option, not further shown in detail, the electronic circuit 14 is provided as a component of at least one of the group of an imaging sensor (e.g. ultrasound, optical), a physiological data measurement sensor (e.g. pressure, volumetric flow, flow velocity), and a rectifier.

In any of the examples, the electronic circuit may be connected to an electrically conductive electrode for providing pacing signal to an anatomy of the body of a subject and/or to ablate the anatomy of the body.

In any of the examples the electronic circuit may be integrated within the first or second sections, and the guidewire is configured to have a smooth, flush outer surface. In a further exemplary embodiment the guidewire has a constant cross section from the proximal end to the distal end.

For example, the electronic circuit is a pressure sensor on the guidewire, e.g. for fractional flow reserve measurement.

In an example, the rectifier is provided to transfer the electromagnetic radiation into DC electric power.

The pressure sensor may be provided as a capacitor, of which capacity is modified by pressure, such that when inserted into a vessel, for example, the resonance frequency is changed, which can then be detected.

The rectifier is provided as a device that can gather electromagnetic energy and that can release such electromagnetic energy. In an example, the rectifier is configured to receive energy.

As indicated above, in an example, an electronic circuit is powered via a rectifier. In an option, the information can thus be sent back. For example, the pressure information may be sent back e.g. by periodically increasing the power dissipation in the chip while the frequency of this dissipation modulation is related to the measured pressure. As the reflected power from the guidewire (and the subject) is modulated, an amplitude modulated total signal can be received by the sending antenna, or by a separate antenna and amplified/demodulated to get the information back from the device.

In an example, not further shown, in addition to the energy supply, a receive path by the same or a different external antenna is provided.

In another example, also not further shown, additional sections are provided in the guidewire without placing chips between the sections.

This results in an increase of the signal at the chip further, e.g. for operation at the higher end of the useful frequency range. A sort of Yagi-(Uda)-Antenna is provided and energy is transferred from one resonant element to the next.

Fig. 2 shows an example of a system 50 for medical intervention. The system 50 comprises a generator 52 for generating electromagnetic radiation. Further, a guidewire 54 according to one of the preceding examples is provided. The generator 52 is configured to generate electromagnetic radiation to be received by the guidewire 54, i.e. by the antenna arrangement of the first section 18 and the second section 20 of the guidewire 54.

In an example, the elongated main body 12 of the guidewire 54 comprises a first end configured as a distal end 56 for insertion into a subject 58, and a second end configured as a proximal end 60, i.e. a handling portion for steering the guidewire inside the subject 58.

The electronic circuit that is arranged on the distal end 56 of the guidewire 54 is supplied by electric energy transferred from the received electromagnetic radiation. As indicated, the guidewire can be used for being inserted into the subject 58, e.g. arranged on a patient support 62. Further, the system 50 for medical intervention may also comprise a medical imaging arrangement 64, which is shown as a C-arm based X-ray imaging device.

The term "subject" may also be referred to as individual. The "subject" may further also be referred to as patient, although it is noted that this term does not indicate whether any illness or disease is actually present with the subject.

In an example, the generator 52 for generating electromagnetic radiation supplies the electromagnetic radiation along the guidewire. In another example, the generator 52 for generating electromagnetic radiation supplies the electromagnetic in the vicinity of a subject, when the guidewire is inserted into the subject.

In an option, not further shown, the generator 52 is configured to generate electromagnetic radiation at an operation frequency that approximately matches with a resonance frequency of the antenna formed by the first section 18 and the second section 20.

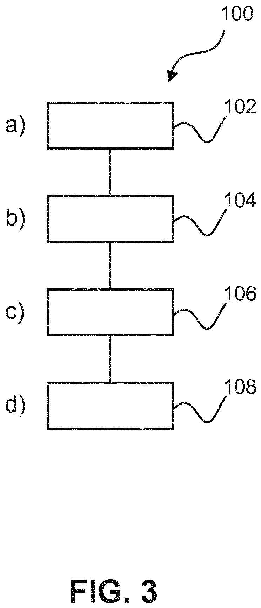

Fig. 3 shows an example of a method 100 for supplying an electronic circuit with energy.

In a first step 102, also referred to as step a), a guidewire for a medical intervention is provided. The guidewire comprises an elongated main body and an electronic circuit. The elongated main body has a longitudinal axis and comprises a first section and a second section arranged in a consecutive manner along the longitudinal axis. The first section and the second section are mechanically connected to each other forming a continuous guide, and are each made from an electrically conductive material, and are separated from each other by an electrically isolating layer such that the first section and the second section act as an antenna for transmitting electric energy to the electronic circuit. The electronic circuit is attached to the elongated main body and is electrically connecting the first section and the second section.

In a second step 104, also referred to as step b), electromagnetic radiation is generated.

In a third step 106, also referred to as step c), the electromagnetic radiation is received by the antenna formed by the first section and the second section and an electric potential is generated.

In a fourth step 108, also referred to as step d), the generated electric potential is supplied to the electronic circuit for operating the electronic circuit.

In an option, for step b), an operation frequency is provided that approximately matches with a resonance frequency of the antenna formed by the first and second section.

The operation frequency is chosen such that the antenna structure operates approximately at the resonance frequency of the antenna. As a result, voltage and thus also energy absorption are maximized. The resonance frequency is depending on the exact geometry of the antenna structure. For example, the resonance frequency is in the range of approximately 100 MHz to 1 GHz. In an example, the structure size of the antenna formed by the guidewire will be tuned in a way that the frequency lies in a permitted band at the location of operation of the guidewire energy supply, e.g. a permitted band of operation in the cathlab or other examination room in a hospital, for example 433 MHz.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail referring to guidewire in the drawings and foregoing description, in alternative embodiments of the device, system and method the guidewire is substituted by one of an interventional device such as intravascular catheter, interventional needle, implantable device for providing physiological measurement data or treatment. The illustrations and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. An interventional device (10) configured for at least partial insertion into a body of a subject for medical intervention, comprising:
- an elongated main body (12); and
- an electronic circuit (14);
wherein the elongated main body has a longitudinal axis (16) and comprises a first section (18) and a second section (20) arranged in a consecutive manner along the longitudinal axis;
wherein the first section and the second section:
- are mechanically connected to each other forming a continuous mechanical structure;
- are each made from an electrically conductive material; and
- are separated from each other by an electrically isolating layer (22);
wherein the electronic circuit is attached to the elongated main body and is electrically connecting the first section and the second section for being supplied by the transmitted electric energy; and
wherein the first section and the second section act as an antenna for transmitting electric energy to/from the electronic circuit.

2. Device according to claim 1, wherein the first section and the second section are configured as a passive antenna provided to receive energy supplied in form of electromagnetic radiation that generates a local electric potential between the first section and the second section; and
wherein the electric potential is supplied as electric energy to the electronic circuit.

3. Device according to claim 1 or 2, wherein the first section and the second section are both provided with a minimum length of 0,1 cm.

4. Device according to claim 1, 2 or 3, wherein the first section is provided as a front tip and the second section is provided as a main shaft portion.

5. Device according to one of the preceding claims, wherein, for the mechanical connection, one of the first section and the second section comprises a front-end reception (24), and the other one of the first section and the second section comprises a front-end extension (26) inserted into the reception.

6. Device according to one of the preceding claims, wherein the first section and the second section are provided with an outer isolating layer.

7. Device according to one of the preceding claims, wherein the electronic circuit is provided as a component of at least one of the group of:
i) a physiological data measurement sensor;
ii) an imaging sensor; and
iii) a rectifier.

8. Device according to one of the preceding claims, wherein, in addition to the energy supply, a receive path by the same or a different external antenna is provided.

9. Device according to one of the preceding claims, wherein additional sections are provided in the device without placing chips between the sections.

10. Device according to one of the preceding claims, wherein the device is a guidewire, and the electrically conductive material comprises metal.

11. A system (50) for medical intervention, comprising:
- a generator (52) for generating electromagnetic radiation; and
- a device (54) according to one of the preceding claims;
wherein the generator is configured to generate electromagnetic radiation to be received by the device; and
wherein the electronic circuit is supplied by electric energy transferred from the received electromagnetic radiation.

12. System according to claim 11, wherein the generator is configured to generate electromagnetic radiation at an operation frequency that approximately matches with a resonance frequency of the antenna formed by the first section and the second section.

13. A method (100) for supplying an electronic circuit with energy, the method comprising the following steps:
a) providing (102) an interventional device configured for at least partial insertion into a body of a subject for a medical intervention;
wherein the device comprises an elongated main body and an electronic circuit; wherein the elongated main body has a longitudinal axis and comprises a first section and a second section arranged in a consecutive manner along the longitudinal axis; wherein the first section and the second section are mechanically connected to each other forming a continuous structure, and are each made from an electrically conductive material, and are separated from each other by an electrically isolating layer such that the first section and the second section act as an antenna for transmitting electric energy to/from the electronic circuit;
wherein the electronic circuit is attached to the elongated main body; and is electrically connecting the first section and the second section;
b) generating (104) electromagnetic radiation;
c) receiving (106) the electromagnetic radiation by the antenna formed by the first section and the second section and generating an electric potential; and
d) supplying (108) the generated electric potential to the electronic circuit for operating the electronic circuit.

14. Method according to claim 13, wherein for step b), an operation frequency is provided that approximately matches with a resonance frequency of the antenna formed by the first section and the second section.
